# EUROPÄISCHE PATENTSCHRIFT

(11) **EP 0 436 261 B1**
(45) Veröffentlichungstag und Bekanntmachung des Hinweises auf die Patenterteilung: **26.05.1993**
(21) Anmeldenummer: 90250330.9
(22) Anmeldetag: 21.12.1990
(51) Int. Cl.: A61K 37/547, C12N 9/72

(54) **Thrombolytisch wirkendes Kombinationspräparat**
Combined preparation with thrombolytic effect
Préparation combinée à l'effet thrombolytique

(30) Priorität: 22.12.1989 DE 3943241
(43) Veröffentlichungstag der Anmeldung: 10.07.1991
(73) Patentinhaber: SCHERING AKTIENGESELLSCHAFT, 13342 Berlin (DE)
(72) Erfinder: Donner, Peter, Dr., W-1000 Berlin 41 (DE); Schleuning, Wolf-Dieter, Dr., W-1000 Berlin 20 (DE)

(56) Entgegenhaltungen:
- EP-A- 0 181 267
- EP-A- 0 383 417

## Beschreibung

Die vorliegende Erfindung betrifft neue Mittel mit thrombolytischer Wirkung.
Thrombosen entstehen durch die Bildung eines Blutgerinnsels in Blutgefäßen. Man unterscheidet venöse Thrombosen einschließlich der Lungenembolien und arterielle Thrombosen einschließlich des akuten Herzinfarkts.

Lungenembolie und Herzinfarkt sind lebensbedrohliche Ereignisse, die einer medizinischen Sofortintervention bedürfen.

Neben verschiedenen invasiven Methoden hat sich in den letzten Jahren die enzymatische Thrombolyse mit Plasminogenaktivatoren als Therapieform für arterielle und venöse Thrombosen durchgesetzt. Diese als Thrombolytika bezeichneten Stoffe wandeln Plasminogen, das inaktive Proenzym des Fibrinolysesystems im Blut, in das aktive proteolytische Enzym Plasmin um. Plasmin wiederum löst den Faserstoff Fibrin, ein wesentlicher Bestandteil eines Blutgerinnsels, auf, was zur Wiedereröffnung der verschlossenen Gefäße und zur Rekonstitution des Blutflusses führt. Plasmin jedoch ist eine relativ unspezifische Protease, das heißt, einmal im Blut gebildet, zerstört sie proteolytisch Komponenten im Blut, die für eine intakte Hämostase unerläßlich sind (z.B. Fibrinogen), und induziert dadurch unter Umständen gefährliche Blutungsrisiken.

Die Thrombolytika der ersten Generation, Streptokinase und Urokinase sind Stoffe, die, einmal in die Zirkulation injiziert, systemisch Plasminogen in Plasmin umwandeln und eine systemische Proteolyse induzieren. Thrombolysetherapien mit diesen Stoffen sind daher häufig von Blutungskomplikationen begleitet. Die daraufhin entwickelte fibrinspezifische Thrombolyse, bei der rekombinierte Plasminogenaktivatoren vom Gewebetyp, kurz t-PA genannt, eingesetzt werden, sollte aus diesem Dilemma herausführen. t-PA hat im Blutkreislauf eine nur geringe Affinität zu Plasminogen. In Gegenwart des Faserstoffs Fibrin jedoch, mit dem es über spezifische Bindungsstellen reagiert, erhöht sich diese Affinität um ein Vielfaches, was in einer Plasminbildung an der Thrombusoberfläche resultiert. Dieses Konzept konnte in vitro und in tierexperimentellen Untersuchungen verifiziert werden; die klinischen Studien ergaben jedoch, daß große Mengen t-PA nötig sind, um die rasche Auflösung einer Koronarthrombose zu bewirken.

Infundiert man aber derart hohe Dosen t-PA, führt das ähnlich wie in den Fällen von Streptokinase und Urokinase zu einer systemischen Proteolyse verbunden mit einem relativen Blutungsrisiko. Man spricht heute von einer relativen Fibrinspezifität des t-PA. Die Ursache dafür liegt in einer wesentlichen Eigenschaft des t-PA begründet: dieses Molekül ist eine Protease, die unter günstigen Bedingungen (hohe Enzymkonzentration, lange Expositionszeit, hohe Substratkonzentration, optimaler pH und Ionenmilieu) Plasminogen auch in Abwesenheit von Fibrin in Plasmin umwandelt. Alle diese Bedingungen sind bei der derzeitigen klinischen Standardtherapie mit t-PA erfüllt.

Bei der Suche nach spezifischeren Plasminogenaktivatoren, die das Kriterium der Fibrinspezifität erfüllen, wurde ein neuer, natürlicher, fibrinolytisch wirksamer Stoff mit der Bezeichnung v-PA (auch DSPA genannt, von Desmodus Salivary Plasminogen Activator) gefunden (EP-A- 0 383 417).

Die Erfindung betrifft ein thrombolytisch wirkendes Mittel bestehend aus einer der nicht-Fibrin-bindenden niedermolekularen Formen DSPA β oder DSPA γ mit den Molekulargewichten 46.000 und 42.000 (EP-A- 0 383 417) und einem Fibrin-bindenden Plasminogenaktivator.

Als Fibrin-bindende Plasminogenaktivatoren kommen z.B. in Betracht: Prourokinase (scu-PA), t-PA und die höhermolekulare Form des Thrombolytikums v-PA (DSPA α1 und DSPA α2, EP-A- 0383 417) mit einem Molekulargewicht von 52.000.

Der thrombolytische Wirkstoff v-PA (DSPA) stellt einen neuen, natürlich vorkommenden Plasminogenaktivator dar, der Blutgerinnsel im menschlichen Körper auflöst und somit zur Behandlung z.B. des Herzinfarkts geeignet ist. (EP-A- 0383 417)

Er findet sich im Speichel aller Spezies von Fledermäusen der Gattung Desmodus spec. in geringen Konzentrationen und wird von den Zellen der Speicheldrüsen dieser Tiergattung exprimiert. Die Fledermäuse der Gattung Desmodus spec. umfassen alle Fledermaus-Arten des amerikanischen Kontinents. Besonders gut wurde die Gattung Desmodus Mittelamerikas und Mexikos untersucht.

Die Erfindung beinhaltet ferner Arzneimittel auf Basis der oben genannten Kombinationen aus nicht-Fibrin- und Fibrin-bindenden Plasminogenaktivatoren sowie üblichen Hilfs- und Trägerstoffen.

Durch die oben beschriebene Kombination fibrinbindender und nicht-fibrinbindender Plasminogenaktivatoren wird die fibrinolytische Potenz von v-PA (DSPA) gegenüber herkömmlichen Plasminogenaktivatoren noch verstärkt. Die Tagesdosis beträgt bei parenteraler Applikation am Menschen 5-500 mg, bevorzugt 20-200 mg.

### BEISPIEL

Fibrinogen und Plasminogen werden in einer Petrischale durch Zugabe von Thrombin zum Gerinnen gebracht. In die entstandene Fibrinschicht werden Löcher von 3 mm Durchmesser gestanzt, in welche nun v-PA in verschiedenen Konzentrationen und Kombinationen gegeben werden. Beide molekularen Formen des fibrinspezifischen Plasminogenaktivators v-PA erzeugen nach Inkubation bei 37°C in einer feuchten Kammer konzentrationsabhängige Lysehöfe.

Kombiniert man nun beide molekular unterschiedlichen v-PA Formen miteinander, so ergeben sich Lysehöfe die deutlich größer sind als die Lysehöfe die man auf Grund eines einfachen additiven Effektes erwarten würde. Durch die Kombination von hoch- und niedermolekularen v-PA erzielt man also in vitro einen synergistischen Effekt der Fibrinolyse. Dieser synergistische Effekt ist darauf zurückzuführen, daß die niedermolekulare Form von v-PA, welcher die Fingerdomäne fehlt, durch ihre nichtfibrinbindende Eigenschaft besser in ein Fibringerinnsel eindiffundieren kann und dort das Gerinnsel durch seine fibrinolytische Aktivität auflockert. Die hochmolekulare fibrinbindende v-PA-Variante kann dann das bereits aufgelockerte Fibringerinnsel vollständig lysieren.

Berechnungen der Ergebnisse dieses Fibrinplattentests haben ergeben, daß durch die Kombination beider v-PA Formen ein eindeutig synergistischer Effekt erzielt wird (Berenbaum, M.C. Clin.Exp. Immunol., 28 , 1-18, 1977).

## Patentansprüche

1. Thrombolytisch wirkendes Mittel bestehend aus der Kombination einer der nicht-fibrinbindenden Formen des Thrombolytikums v-PA DSPA β oder DSPA γ mit den Molekulargewichten 46.000 und 42.000 und einem fibrin-bindenden Plasminogenaktivator sowie üblichen Hilfs- und Trägerstoffen.

2. Thrombolytisch wirkendes Mittel nach Anspruch 1, dadurch gekennzeichnet, daß Prourokinase (scu-PA), t-PA oder die höhermolekularen Formen des Thrombolytikums v-PA (DSPA α1 und DSPA α2) mit einem Molekulargewicht von 52.000 den Fibrin-bindenden Plasminogenaktivator darstellen.

## Claims

1. Thrombolytically active agent consisting of a combination of one of the non-fibrin-binding forms of the thrombolytic agent v-PA, DSPA β or DSPA γ, with molecular weights of 46,000 and 42,000, and a fibrin-binding plasminogen activator, and also conventional excipients and carriers.

2. Thrombolytically active agent according to claim 1, characterised in that prourokinase (scu-PA), t-PA or the high-molecular-weight forms of the thrombolytic agent v-PA (DSPA α1 and DSPA α2) having a molecular weight of 52,000 comprise the fibrin-binding plasminogen activator.

## Revendications

1. Produit thrombolytique constitué de l'association de l'une des formes ne fixant pas la fibrine du thrombolytique v-PA, DSPA β et DSPA γ, de masses moléculaires respectives 46 000 et 42 000, et d'un plasminogène-activateur fixant la fibrine, avec des adjuvants et véhicules habituels.

2. Produit thrombolytique selon la revendication 1, caractérisé en ce que le plasminogène-activateur fixant la fibrine est la pro-urokinase (scu-PA), le t-PA ou les formes à plus haute masse moléculaire du thrombolytique v-PA (DSPA α1 et DSPA α2) de masse moléculaire 52 000.
